# EUROPEAN PATENT APPLICATION

(11) **EP 4 810 473 A1**
(43) Date of publication of application: **23.09.2026**
(21) Application number: 25165094.1
(22) Date of filing: 20.03.2025
(51) Int. Cl.: C07C 45/50, C07C 47/02, F25B 30/06

(54) **PROCESS FOR PRODUCING BUTYRALDEHYDE**

(71) Applicant: BASF SE, 67056 Ludwigshafen am Rhein (DE)
(72) Inventor: CLAUSEN, Iven, 67056 Ludwigshafen am Rhein (DE); KUMAR ARORA, Sachin, 67056 Ludwigshafen am Rhein (DE); KIRCHNER, Tanja, 67056 Ludwigshafen am Rhein (DE)
(74) Representative: Kudla, Karsten

(57) **Abstract**

The invention relates to a process for producing butyraldehyde, comprising:
(a) feeding propene (1) and a hydrogen and carbon monoxide containing synthesis gas (3) into a reactor (5);
(b) reacting propene, hydrogen and carbon monoxide to obtain an isobutyraldehyde and n-butyraldehyde comprising crude product stream (7);
(c) withdrawing the crude product stream (7) from the reactor (5);
(d) cooling the crude product stream (7);
wherein the crude product stream (7) is cooled in a heat exchanger (201) of a heat pump (200) for producing steam and/or in a heat exchanger (101) of a district heating grid (100).

## Description

The invention relates to a process for producing butyraldehyde, comprising:
(a) feeding propene and a hydrogen and carbon monoxide containing synthesis gas into a reactor;
(b) reacting propene, hydrogen and carbon monoxide to obtain an isobutyraldehyde and n-butyraldehyde comprising crude product stream;
(c) withdrawing the crude product stream from the reactor;
(d) cooling the crude product stream.

Butyraldehyde is an important compound that is used for example in the production of additives for polymers like vulcanization accelerators or plasticizer. Further, butyraldehyde is used for producing n-butanol in industrial scale.

For obtaining n-butyraldehyde and isobutyraldehyde, the crude product stream is worked up. In a first stage, gaseous components, particularly carbon-monoxide and hydrogen, and non-reacted propene are removed. In a following distillation process, the n-butyraldehyde and iso-butyraldehyde are separated, wherein n-butyraldehyde is obtained as bottom product and iso-butyraldehyde as top product of a distillation column.

The process for producing butyraldeyde is described for example in Donald K. Raff, "Butanals", Ullmann's Encyclopedia of Industrial Chemistry, 2013.

For cooling the crude product stream, usually a cooler operated with air or water as cooling medium is used. However, using air or a water as cooling medium has the disadvantage that heat of the crude product stream is wasted.

Therefore, it was an object of the present invention to provide a process for producing butyraldehyde, in which less heat is wasted by removing it with a cooling medium.

This object is achieved by a process for producing butyraldehyde, comprising:
(a) feeding propene and a hydrogen and carbon monoxide containing synthesis gas into a reactor;
(b) reacting propene, hydrogen and carbon monoxide to obtain an isobutyraldehyde and n-butyraldehyde comprising crude product stream;
(c) withdrawing the crude product stream from the reactor;
(d) cooling the crude product stream;
wherein the crude product stream is cooled in a heat exchanger of a heat pump for producing steam and/or in a heat exchanger of a district heating grid.

By cooling the isobutyraldehyde and n-butyraldehyde comprising crude product stream in the heat exchanger of the heat pump for producing steam or in the heat exchanger of a district heating grid, heat of the crude product stream that usually is discarded by using air or cooling water for cooling the crude product stream, is used as energy source for producing steam or as energy source in the district heating grid. By using the heat of the crude product stream as energy source for producing steam or as energy source in the district heating grid, the amount of fossil based energy that usually is used as energy source for producing steam or in the district heating grid can be reduced. By this reduction, also the carbon dioxide footprint of steam generation or heat generation for the district heating grid is reduced.

In the context of the present invention, the term "butyraldehyde" means n-butyraldehyde and/or isobutyraldehyde and particularly any mixture of n-butyraldehyde and isobutyraldehyde.

For producing butyraldehyde, propene and a hydrogen and carbon monoxide containing synthesis gas are fed into a reactor, in which the propene reacts with the hydrogen and carbon monoxide in the presence of a catalyst, thereby forming an isobutyraldehyde and n-butyraldehyde comprising crude product stream.

The synthesis gas fed into the reactor usually contains 33 to 67 mol-% hydrogen and 33 to 67 mol-% carbon monoxide, more preferred 45 to 55 mol-% hydrogen and 45 to 55 mol-% carbon monoxide.

The ratio of propene to synthesis gas preferably is in a range from 1 : 2.0 to 1 : 3.0, more preferred in a range from 1 : 2.0 to 1 : 2.6, and particularly in a range from 1 : 2.0 to 1 : 2.3.

Generally, the reaction is carried out at a temperature in a range from 60 to 130 °C, more preferred in a range from 80 to 125 °C and particularly in a range from 90 to 120 °C and a pressure in a range from 1 to 30 bar(abs), more preferred in a range from 5 to 30 bar(abs) and particularly in a range from 10 to 25 bar(abs). The catalyst may be any catalyst known to a skilled person that is suitable for being used for producing butyraldehyde by reacting propene, hydrogen and carbon monoxide. Suitable catalysts for example are rhodium and cobalt based, particularly rhodium based.

From the reactor, the isobutyraldehyde and n-butyraldehyde comprising crude product stream is withdrawn. The crude product stream generally is cooled to a temperature in a range from 60 to 125 °C, more preferred to a temperature in a range from 80 to 110 °C and particularly to a temperature in a range from 100 to 110 °C. By cooling the crude product stream, at least a part of the butyraldehyde contained in the crude product stream condenses.

After cooling, the crude product stream preferably is fed into a vessel for separating gaseous components from crude butyraldehyde. Since due to cooling, at least a part of the butyraldehyde is condensed, separation of the gaseous components from the crude butyraldehyde preferably is carried out by phase separation. The crude butyraldehyde is withdrawn from the vessel for separating the gaseous components from the crude butyraldehyde, is heated and then fed into a work-up unit. Preferably, the crude butyraldehyde withdrawn from the vessel is heated in an internal heat exchanger by heat transfer from the crude product stream before being fed into the work-up unit.

In the work-up unit, butyraldehyde is separated from low boilers, for example non reacted gaseous components, particularly hydrogen, carbon monoxide and propene, which remained solved in the crude butyraldehyde and propane, that may be obtained as a byproduct by reaction of propene with hydrogen. In a following separation unit, the propane and non-reacted propene are separated from hydrogen and carbon monoxide and the hydrogen and carbon monoxide are recycled into the reaction.

The mixture containing propene and propane is fed into a separation column, in which the propene is distilled off the mixture. The propene, which is obtained as top stream is withdrawn and returned into the reaction and the propane may be withdrawn as an additional product and used for example as a fuel.

The gaseous components withdrawn from the vessel for separating the gaseous components from the crude butyraldehyde preferably are returned into the reaction. Since the crude reaction product was cooled, it is preferred that the gaseous components are heated before being returned into the reaction. Particularly preferably, the gaseous components are heated in an internal heat exchanger by heat transfer from the crude product stream before being recycled into the reactor.

If the crude butyraldehyde and the gaseous components are heated in internal heat exchangers by heat transfer from the crude reaction product, it is preferred that the crude reaction products flows through the internal heat exchanger for heating the crude butyraldehyde and the internal heat exchanger for heating the gaseous components in series, particularly that the crude reaction product firstly flows through the internal heat exchanger for heating the crude butyraldehyde and subsequently through the internal heat exchanger for heating the gaseous components. However, besides arranging the internal heat exchangers such that the crude reaction product flows through the internal heat exchangers in series, it is also possible that the internals heat exchangers are arranged such that the crude reaction product is separated in two partial streams that flow through the internal heat exchangers in parallel.

By heating the crude butyraldehyde and/or the gaseous components by heat transfer from the crude reaction product, the crude reaction product is cooled. For minimizing the heat to be additionally supplied for heating the crude butyraldehyde and/or the gaseous components, it is preferred that the crude product stream is cooled in the internal heat exchanger for heating the crude butyraldehyde and/or the internal heat exchanger for heating the gaseous components before being cooled in the heat exchanger of the heat pump.

By heat transfer for heating the crude butyraldehyde and/or the gaseous components, the crude reaction product is cooled to a temperature in a range from 70 to 110 °C particularly in a range from 80 to 100 °C. Since the crude reaction product cooled in the internal heat exchanger for heating the crude butyraldehyde and/or the internal heat exchanger for heating the gaseous components still contains a large amount of heat, according to the present invention, this heat is used for producing steam and/or in a district heating grid.

If the crude product stream is cooled in the heat exchanger of the heat pump for producing steam, the heat pump may be an open loop heat pump or a closed loop heat pump. Preferably, the heat pump is a closed loop heat pump.

In an open loop heat pump, water is heated in the heat exchanger by heat transfer from the crude product stream. In the heat exchanger, the water may be at least partly evaporated. Alternatively, the heated water may be fed into a flash apparatus and at least partly evaporated by expansion. If only a part of the water is evaporated in the heat exchanger or in the flash apparatus, the gaseous phase is separated from the liquid phase and withdrawn as steam and the liquid phase may be returned into the heat exchanger to be heated and, optionally, partly evaporated, or may be withdrawn and used as heated water. However, preferably, the liquid phase is returned into the heat exchanger.

If a flash apparatus is used, the flash apparatus may be any apparatus suitable for flash evaporation and subsequent phase separation. Preferably, the flash apparatus is a flash tank, in which saturated steam forms an upper phase and the water not being evaporated a lower liquid phase. The pressure in the flash tank may be set by using a vacuum pump or, preferably, by a compressor used for compressing the steam. The water withdrawn from the flash apparatus and recycled into the heat exchanger is compressed before being fed into the heat exchanger. As some of the water is withdrawn as steam, it is necessary that also fresh water is fed into the heat exchanger. Preferably, the fresh water and the recycled water are mixed and then fed into the heat exchanger. The fresh water and the recycled water may be mixed before being compressed in the pump or, if the pressure of the fresh water already corresponds to the pressure with which the water is to be fed into the heat exchanger, downstream the pump for compressing and feeding the recycled water into the heat exchanger. The fresh water fed into the heat exchanger preferably is degassed and demineralized water.

The water being fed into the heat exchanger of the open loop pump preferably has a pressure in a range from 1 to 4 bar(abs), more preferred in a range from 1.5 to 2.5 bar(abs) and particularly in a range from 1.9 to 2.2 bar(abs). The temperature of the water being fed into the heat exchanger preferably is in a range from 30 to 70 °C, more preferred in a range from 40 to 70 °C and particularly in a range from 55 to 65 °C.

In the heat exchanger, the water preferably is heated to a temperature in a range from 45 to 95 °C, more preferred to a temperature in a range from 55 to 85 °C and particularly to a temperature in a range from 65 to 75 °C.

For evaporating, the water stream is expanded in the flash apparatus to a pressure in a range from 0.01 to 1.0 bar(abs), more preferred to a pressure in a range from 0.1 bar(abs) and particularly to a pressure in range from 0.15 to 0.4 bar(abs).

Besides using an open loop heat pump with a flash apparatus, it is also possible that the open loop heat pump comprises a heat exchanger, in which a water stream is evaporated by heat transfer from the crude product stream, and a compressor, in which the evaporated water is compressed to generate process steam. If no flash evaporator is used, it is necessary that the water being heated in the heat exchanger has pressure being low enough that at least a part of the water evaporates in the heat exchanger by heat transfer from the crude product stream. Preferably, the water being fed into the heat exchanger has a pressure in a range from 0.05 to 0.85 bar(abs), more preferred in a range from 0.15 to 0.6 bar(abs) and particularly in a range from 0.2 to 0.4 bar(abs).

The temperature, with which the water is fed into the heat exchanger of the open loop heat pump preferably corresponds to the temperature of the water being fed into the heat exchanger of the heat pump with flash apparatus.

In a closed loop heat pump, which is preferred, by cooling the product stream in the heat exchanger of the heat pump, a working medium is evaporated by heat transfer from the product stream. After being evaporated, the working medium is compressed. By compression, the temperature of the working medium increases. Subsequently, the working medium passes a second heat exchanger, in which heat is transferred to water. After being cooled by heat transfer to heat the water, the working medium is expanded and thereby further cooled.

The water may be at least partly evaporated in the second heat exchanger. Alternatively, the water is heated and subsequently fed into a flash apparatus, in which the water is expanded and by expanding, at least a part of the water evaporates. If only a part of the water is evaporated, the liquid and the gaseous phase are separated, the gaseous phase is withdrawn as steam and the liquid phase is recycled into the second heat exchanger for being heated and optionally at least partly evaporated.

If the water is evaporated completely in the second heat exchanger, no phase separation needs to take place.

The working medium used in the closed loop of the closed loop heat pump may be any suitable working medium, for example water, glycol-water mixtures or heat transmission liquids like ethyleneoxide, cyclobutene, cis-butene, trans-butene, 1,3-butadiene, 1-butene, butane, isobutene, dimethylether, cyclopropane, ammonia, propandiene, neopentane, isobutane, R441A, R1233ZDE, R1234ZEZ, R1224YDZ, R40, R436B, R510A, R435A, R429A, RE235CB2, R152A and R440A.

The water being evaporated in the second heat exchanger of the closed loop heat pump preferably has a pressure in a range from 1 to 5 bar(abs), more preferred in a range from 1.1 to 2.5 bar(abs) and particularly in a range from 1.2 to 1.8 bar(abs).

The steam generated in the heat pump may be used directly without further compression, if the temperature of the steam is sufficiently high to be used as an energy source in a process, for example for heating a stream or an apparatus. Processes that may use the steam as energy source may be any processes known to a skilled person that must be heated. The steam may be used, for example, for heating a reactor or for providing heat in a distillation process.

Besides directly using the steam for heating a stream or an apparatus, it is also possible to feed at least a part of the steam into a steam grid, which provides heat for several processes.

Depending on the temperature of the steam and the intended use, it may be necessary to compress the steam to obtain compressed steam. Compressing the steam particularly may be necessary if the steam is not used directly in a process but fed into a steam grid. Further, if the steam is obtained by expansion in a flash apparatus, or if the steam is obtained by evaporation in the heat exchanger of the open loop heat pump, the pressure and, hence, the temperature of the steam generally is too low to use the steam directly, so that in these cases the steam generally is compressed before being used.

The compressed steam may be low pressure steam, medium pressure steam, or high pressure steam. Further, it is possible to divide the steam produced in the heat pump into separate streams which can be compressed to different pressures. This allows, for example, to produce medium pressure steam and high pressure steam, low pressure steam and medium pressure steam, low pressure steam and high pressure steam, or even low pressure steam, medium pressure steam and high pressure steam.

In the context of the present invention, the term "low pressure steam" means steam having a pressure in a range from 0.9 to 4 bar(abs), more preferred in a range from 1 to 2 bar(abs) and particularly in a range from 1.2 to 1.5 bar(abs), and a temperature in a range from 96 to 160 °C, more preferred in a range from 99 to 140 °C, and particularly in a range from 104 to 120 °C.

The term "medium pressure steam" means steam having a pressure in a range from 4 to 8 bar(abs), more preferred in a range from 4.5 to 7 bar(abs) and particularly in a range from 5 to 6 bar(abs), and a temperature in a range from 143 to 220 °C, more preferred in a range from 147 to 210 °C, and particularly in a range from 151 to 200 °C.

The term "high pressure steam" means steam having a pressure in a range from 8 to 40 bar(abs), more preferred in a range from 10 to 30 bar(abs) and particularly in a range from 16 to 20 bar(abs), and a temperature in a range from 170 to 280 °C, more preferred in a range from 180 to 260 °C, and particularly in a range from 201 to 240 °C.

Particularly preferably, the compressed steam has a pressure in a range from 1 to 25 bar(abs), more preferred a pressure in a range from 4 to 16 bar(abs), and particularly in a range from 5.8 to 7 bar(abs) and a temperature in a range from 100 to 250 °C, more preferred in a range from 145 to 215 °C, and particularly in a range from 160 to 200 °C.

If the steam needs to be compressed, compression may be carried out in any compressor known to a skilled person that is suitable for compressing steam. The compression may be carried out in only one compressor or in a cascade of at least two compression stages. More than one compression stage can be implemented within one compressor comprising more than one compression stage or in a cascade of more than one compressor comprising one or more than one compression stages each. Suitable compressors for example are integrally geared turbo compressors, radial compressors, axial compressors, screw compressors, and piston compressors.

For increasing the energy efficiency and setting the properties of the steam, particularly the temperature, it is possible to inject water before or after at least one compressor or compression stage. Further, by injecting water, the steam is cooled and particularly between two compression stages, cooling the steam has the advantage that damages due to the high temperature of overheated steam are prevented. The water is preferably injected after each compressor or compression stage. Depending on the pressure of the steam into which the water is injected, the water may need to be compressed to a pressure above the pressure of the steam, before being injected. Besides setting the properties of the steam, it is a further advantage that by injection of water the mass flow of steam increases.

The steam produced in the heat pump and the optional subsequent compression can be used in any process, which uses steam having the pressure of the steam generated in the heat pump or by compression. The steam may be used for example as an energy source in the process for producing butyraldehyde or in a process for working up the crude product stream. Further, the steam may also be used in a process that processes the butyraldehyde, for example in a process for producing butanol. Further, the steam also may be used in any other process which preferably is carried out on the industrial site, the process for producing butyraldehyde is carried out. Besides using the steam directly in a process, preferably on the same industrial site, the steam may be fed into a steam grid.

By heating the water in the heat exchanger of the heat pump, the crude product stream preferably is cooled to a temperature in a range from 30 to 85 °C, more preferred in a range from 40 to 75 °C, and particularly in a range from 45 to 70 °C.

To further cool the crude product stream, or to cool the crude product stream in cases where the heat pump is shut off, for example for maintenance purposes, it is preferred to place a cooler downstream the heat exchanger of the heat pump.

Alternatively or additionally, the heat of the crude product stream may be used for a district heating grid. For this purpose, the crude product stream is cooled in a heat exchanger of the district heating grid.

By being cooled in the heat exchanger, the crude product stream may transfer heat to a heating medium that is used in the district heating grid, for example, for providing heat for heating buildings or generating hot water.

As in the embodiment described above, where the heat of the crude product stream is used for producing steam in a heat pump, also when using the heat of the crude product stream in a district heating grid, it is preferred that a cooler is provided downstream the heat exchanger of the district heating grid to further cool the crude product stream, or to cool the crude product stream in cases where less heat is used in the district heating grid, for example in summer.

However, due to the temperature of the crude product stream, heat transfer from the crude product stream will not be sufficient for heating the heating medium of the district heating grid to a temperature that is sufficiently high, and additional heating will be necessary. For this purpose, it is preferred that by being cooled in the heat exchanger, the crude product stream transfers heat to a heat transfer medium that is heated in the heat exchanger of the district heating grid. Subsequently, the heating medium of the district heating grid in a first step is heated by heat transfer from the heat transfer medium and subsequently by additional heating.

The heat transfer medium is used for transferring the heat over the borders of the industrial site where the butyraldehyde is produced to avoid pipes through which butyraldehyde flows to cross the borders, since the district heating grid generally will be outside the industrial site.

If, however, the district heating grid is on the industrial site, for example for heating office buildings or other buildings on the industrial site, or the district heating grid is close to the industrial site, it is possible to transfer the heating medium on the industrial site and transfer heat from the crude product stream to the heating medium in a heat exchanger that is located on the industrial site.

Since district heating grids usually are used for providing heat to residential areas or work areas outside the industrial site on which the butyraldehyde is produced, it is preferred that a heat transfer medium is heated in the heat exchanger of the district heating grid and the heat transfer medium then is used as a heat source for heating the heating medium that is distributed in the district heating grid. The heat transfer medium may be any suitable heat transfer medium, preferably, the heat transfer medium is water.

Particularly if the heat transferred to the heating medium that is distributed in the district heating grid is not sufficient to heat the heating medium to a predefined temperature, it is necessary to provide additional heat for heating the heating medium. For this purpose, it is for example possible that the heat transfer medium is further heated and then used as heating medium in the district heating grid. For further heating, the heat transfer medium may be heated for example in a heater. Alternatively, if a heat transfer medium is used that at least partly evaporates by heat transfer from the crude product stream, further heating may be carried out by compressing the heat transfer medium before it transfers heat to the heating medium, wherein due to compression, the temperature of the heat transfer medium increases. In this case, the heat transfer medium corresponds to the working medium of a closed loop heat pump. After having transferred heat to the heating medium, the heat transfer medium is expanded, wherein due to expansion, the temperature decreases and the thus additionally cooled and expanded heat transfer medium then is recycled into the heat exchanger of the district heating grid in which the heat transfer medium is heated again by heat transfer from the crude product stream.

Alternatively and preferably, the heating medium may be heated in a heat pump by using heat of the heat transfer medium or may be heated by heat transfer from the heat transfer medium in a first heat exchanger and subsequent heating in a heater. Heating the heating medium in a heat pump or in a heater has the advantage, that water can be used as heat transfer medium, which, for safety reasons, is preferred.

If not the heat transfer medium is used as working medium of a heat pump but an additional heat pump is used for heating the heating medium, it is preferred to use a closed loop heat pump with a suitable working medium. In a first heat exchanger of the closed loop heat pump, the working medium is heated and at least partly evaporated, then the working medium is compressed. After compression, the working medium transfers heat to the heating medium, whereby the working medium is cooled. Subsequently, the working medium is expanded and returned into the first heat exchanger of the closed loop heat pump.

If, on the other hand, a heater is used for additionally heating the heating medium, any suitable heater can be used. Suitable heaters for example are electrical heaters, or heaters using heating oil, water or steam as heating medium.

Independent of being cooled by heat transfer in a heat exchanger of a heat pump or in a heat exchanger of a district heating grid, it is preferred that the crude product stream is cooled to a temperature in a range from 20 to 50 °C, more preferred in a range from 25 to 45 °C, and particularly in a range from 30 to 40 °C, before being further processed. For cooling the crude product stream to such a temperature, cooling in the heat exchanger of the heat pump and/or the heat exchanger of the district heating grid may be sufficient. However, if the temperature of the crude reaction product is higher after having left the heat exchanger of the heat pump and/or the heat exchanger of the district heating grid, the additional cooler is used.

If the heat of the crude reaction product is used for producing steam in a heat pump and as heat source in the district heating grid, it is possible to arrange the heat exchanger such that the crude reaction product flows through the heat exchangers in series or in parallel.

Examples of the invention are shown in the figures and described in more detail in the following description.

In the figures:
- Figure 1: shows a process for producing butyraldehyde according to the invention;
- Figure 2: shows a concept of using heat of the crude product stream as heat source in a district heating grid;
- Figure 3: shows a concept of using heat of the crude product stream as heat source in a heat pump for producing steam;

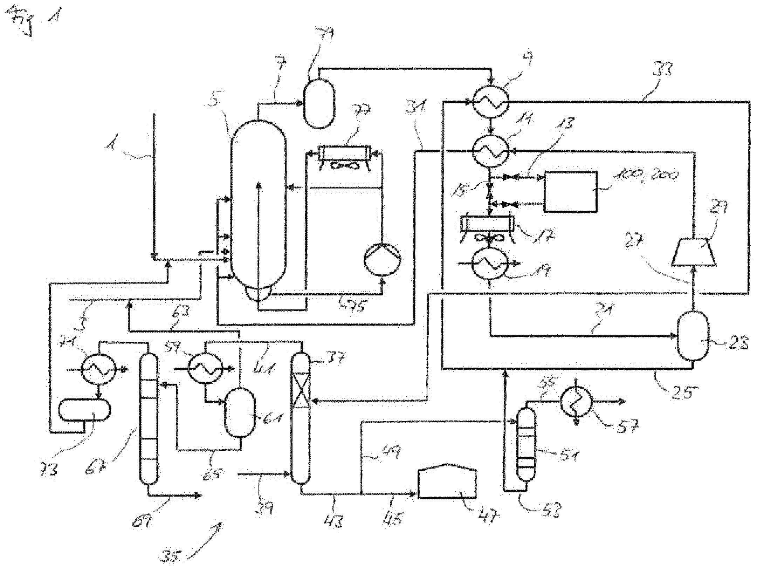

A process for producing butyraldehyde is shown in figure 1.

For producing butyraldehyde, propene 1 and a hydrogen and carbon monoxide comprising synthesis gas 3 are fed into a reactor 5. In the reactor 5, the propene reacts with hydrogen and carbon monoxide, thereby forming n-butyraldehyde and isobutyraldehyde in the presence of a catalyst. The reaction preferably is carried out at a temperature in a range from 60 to 130 °C and a pressure in a range from 1 to 30 bar(abs).

At the top of the reactor 5, a crude product stream 7 is withdrawn. The crude product stream is cooled in a first internal heat exchanger 9 and a second internal heat exchanger 11. After being cooled in the first internal heat exchanger 9 and the second internal heat exchanger 11, at least a part 13 of the crude product stream 7 is used as a heat source in a district heating grid 100 or in a heat pump 200 for producing steam. If only a part 13 of the crude product stream is used as heat source in the district heating grid 100 or the heat pump 200, the part 13 of the crude product stream 7 is remixed with the remaining part 15 of the crude product stream 7.

Depending on the temperature of the crude product stream 7 after being remixed or, if the whole crude product stream 7 is used as the heat source, after passing a heat exchanger of the district heating grid 100 or the heat pump 200, or, if, for example in cases of maintenance, the whole crude product stream 7 bypassed the district heating grid or the heat pump, after having passed the second internal heat exchanger 11, the crude product stream may be cooled in a cooler 17. Subsequently, the crude product stream passes a condenser 19, in which butyraldehyde is condensed from the crude product stream. The cooler 17 for example may be an air cooler as shown here.

The partly condensed crude product stream 21 is fed into a vessel 23 for phase separation and separated into crude butyraldehyde 25 and gaseous components 27 comprising non-reacted reactants, particularly hydrogen and carbon monoxide.

The gaseous components 27 are withdrawn from vessel 23 for phase separation, compressed in a compressor 29 and fed into the second internal heat exchanger 11. By compression, the temperature of the gaseous components increases, remains, however, below the temperature of the crude product stream 21 being fed into the second internal heat exchanger 11. In the second internal heat exchanger 11, the gaseous components 27 are heated by heat transfer from the crude product stream 7. After having passed the second internal heat exchanger 11, the heated gaseous components 31 are recycled into the reactor 5.

The crude butyraldehyde 25, which is withdrawn from vessel 23 for phase separation is passed through the first internal heat exchanger 9. In the first internal heat exchanger 9, the crude butyraldehyde 25 is heated by heat transfer from the crude product stream 7, which simultaneously is cooled. The heated crude butyraldehyde 33 is passed into a work-up section 35.

In the work-up section, particularly C₃-hydrocarbons like propene and propane are removed from the heated crude butyraldehyde 33. For this purpose, the heated crude butyraldehyde 33 is fed into a stripping column 37. For stripping C₃-hydrocarbons, hydrogen and carbon monoxide containing synthesis gas 39 is fed into the stripping column 37 and brought into contact with the heated crude butyraldehyde. By stripping, a gas stream 41 enriched in C₃-hydrocarbons and a crude butyraldehyde with reduced content in C₃-hydrocarbons 43 are obtained. A first part 45 of the crude butyraldehyde with reduced content in C₃-hydrocarbons is collected in a container 47 and a second part 49 of the crude butyraldehyde with reduced content in C₃-hydrocarbons is fed into a column 51 for removing low boilers. From the column 51 for removing low boilers, a liquid butyraldehyde comprising stream 53 is withdrawn, mixed with the crude butyraldehyde 25 and then fed into the first internal heat exchanger 9. The low boilers, particularly hydrogen and carbon monoxide are withdrawn as exhaust gas 55, cooled in an exhaust gas cooler 57 and removed from the process. Particularly preferably, the exhaust gas is used in a process for producing methanol.

The gas stream 41 enriched in C₃-hydrocarbons is cooled in a heat exchanger 59. By cooling in the heat exchanger 59, C₃-hydrocarbons condense. Subsequently, the gas stream with C₃-hydrocarbons being condensed is fed into a second phase separator 61, in which gaseous components 63, particularly carbon monoxide and hydrogen, are separated off. The gaseous components 63 are mixed into the hydrogen and carbon monoxide comprising synthesis gas 3 and fed into the reactor 5.

By separating off the gaseous components 63, a liquid C₃-hydrocarbons comprising stream is obtained, which is fed into a column 67. In the column 67, the liquid C₃-hydrocarbons comprising stream is separated into propane and propene. The propane is withdrawn as bottom stream 69 and collected for further use. The propene is condensed in a propene condenser 71, collected in a buffer container 73 and recycled into the reactor 5. For this purpose, it is particularly preferred to mix the propene withdrawn from the buffer container 73 with the propene 1, which is freshly fed into the process.

Before being fed into the reactor, the propene may be purified to remove traces of substances that might deactivate the catalyst.

For cooling the reaction mixture in the reactor 5, a stream 75 is withdrawn from the reactor, cooled in a cooler 77 and returned into the reactor 5. The cooler 77 may be, for example, an air cooler as shown here.

Since catalyst droplets may be entrained with the crude product stream 7, the crude product stream 7 is passed through a demister 79, in which droplets are separated from the gaseous crude product stream 7.

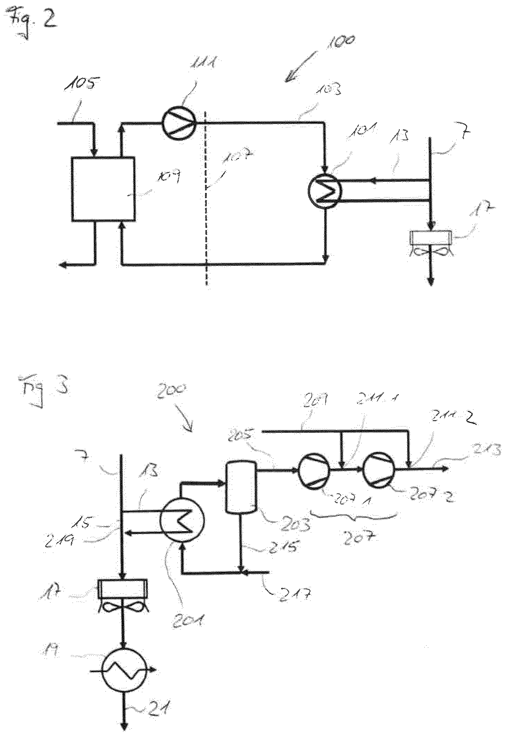

Figure 2 shows a concept of using heat of the crude product stream as heat source in a district heating grid.

If the crude product stream 7 is used as heat source in a district heating grid 100, the crude product stream is fed into a heat exchanger 101 of the district heating grid. In the heat exchanger 101 of the district heating grid, a heat transfer medium 103 is heated by heat transfer from at least a part 13 of the crude product stream 7.

Depending on the area in which the heat of the district heating grid is to be used, the heat transfer medium 103 may be used as heating medium of the district heating grid or the heat transfer medium 103 may be used as a heat source for heating a heating medium 105 as shown here. The heat transfer medium 103 is used as a heat source particularly if the district heating grid is outside the industrial site on which the crude product stream 7 is obtained. This is indicated in figure 2 by a dashed line 107 which symbolizes a border of an industrial site.

If the district heating grid 100 is outside the industrial site as shown here, it is important that no compounds that may be dangerous to animals or human beings or that may be hazardous for the environment pass the border 43 of the industrial site. Also if the district heating grid is used for heating buildings on the industrial site, any compounds that may be dangerous should not be transported along large distances. Therefore, the heat transfer medium preferably is any liquid that is not dangerous for animals or human beings or hazardous for the environment. Particularly preferably, the heat transfer medium is water.

Depending on the distance of the district heating grid to the heat exchanger 101 of the district heating grid 100, the heat transfer medium 103 may be used as heating medium. In this case, it may be necessary to additionally heat the heat transfer medium in a heat exchanger to provide sufficient heat for heating buildings connected to the district heating grid 100.

Alternatively, the heat transfer medium 103 transfers heat to the heating medium 105 as shown here. However, also in this case it may be necessary to supply additional heat for heating the heating medium 105 to a temperature that is sufficiently high for heating or providing hot water in the buildings that are connected to the district heating grid 100.

For supplying additional heat, any suitable device 109 for supplying heat may be used. The device 109 for supplying additional heat may comprise for example a first heat exchanger in which the heating medium 105 is heated by heat transfer from the heat transfer medium 103 and subsequently, the heating medium 105 may be heated in a heater. The heater may be any suitable heater, for example an electrical heater or a heater which is operated with steam.

As an alternative, the device 109 for supplying additional heat may be a heat pump. The heat pump preferably is a closed loop heat pump. In this case, the heat transfer medium transfers heat to a working medium of the heat pump wherein at least a part of the working medium evaporates. The at least partly evaporated working medium is compressed and thereby further heated. Subsequently, the working medium is cooled by heat transfer to the heating medium 105, wherein the heating medium 105 is heated. The cooled working medium then is expanded and recycled into the heat exchanger in which the working medium is at least partly evaporated by heat transfer from the heat transfer medium.

After being used as a heat source for heating the heating medium 105, the heat transfer medium 103 is recycled into the heat exchanger 101 of the district heating grid 100. For circulating the heat transfer medium 103, a pump 111 may be used.

Figure 3 shows a concept of using heat of the crude product stream as heat source in a heat pump for producing steam.

After being cooled in the first internal heat exchanger 9 and the second internal heat exchanger 11, at least a part 13 of the crude product stream 7, preferably the whole crude product stream 7, flows through a heat exchanger 201 of the heat pump 200 for further cooling. In the embodiment shown here, the heat pump 200 is an open loop heat pump with a flash apparatus 203.

In the open loop heat pump, water is heated in the heat exchanger 201 of the heat pump by heat transfer from the at least part 13 of the crude product stream 7. The water then is fed into the flash apparatus 203, in which the water is expanded to a pressure below the boiling pressure of the heated water. By expansion, a part of the water fed into the flash apparatus 203 evaporates, thereby forming saturated steam. The saturated steam 205 is withdrawn from the flash apparatus 203 and compressed in at least one compressor 207. The at least one compressor 207 may be a compressor comprising at least two compression stages 207.1, 207.2. Alternatively, it is also possible to use more than one compressor 207. If more than one compressor 207 is used, it is possible to use compressors which each have at least two compression stages, to use compressors that each have only one compression stage or at least one compressor having at least two compression stages and at least one compressor having one compression stage.

Since the steam is heated by compression, generally water 209 is fed into the steam downstream of at least one compression stage 207.1, 207.2. The water being fed into the steam evaporates, thereby cooling the steam. Further, by feeding the water, the amount of steam increases. To avoid damage of a compression stage 207.1, 207.2, each injection point 211.1, 211.2 of water is followed by an inlet path having a length that is sufficient for evaporating the whole water that was injected at the injection point 211.1, 211.2 before the steam enters the following compression stage 207.2.

After compression, compressed steam 213 is withdrawn from the last compression stage 207.2 as process steam. The compressed steam 213 may be used for example as an energy source in a process for working up a raw product stream obtained in a process for producing butanol. The water that is not evaporated in the flash apparatus 203 is withdrawn as liquid phase 215 and preferably recycled into the heat exchanger 201 of the heat pump 200. To obtain continuous conditions in the heat pump 200, the part of the water that is withdrawn as saturated steam 205 needs to be replaced. For this purpose, fresh water 217 is fed into the liquid phase 215 recycled into the heat exchanger 201. The fresh water usually is demineralized and degassed water, for example boiler feed water.

To provide the possibility to only feed the part 13 of the crude product stream 7 into the heat exchanger 201 of the heat pump 200 or to bypass the heat exchanger 201 of the heat pump 200, if the heat pump may be shut off, for example for maintenance purposes, a bypass 219 is provided.

If only a part 13 of the crude product stream 7 passes the heat exchanger 201, the remaining part 15 of the crude product stream 7 flows through the bypass 219. After having passed the heat exchanger 201, the part 13 of the crude product stream 7 is recombined with the remaining part 15 of the crude product stream 7 that passed the bypass 219. To allow further cooling of the crude product stream 7, the cooler 17 may be provided. The cooler 17 particularly is used, if the crude product stream 7 still has a temperature being too high for being fed into the condenser 19, after being cooled by heat transfer in the heat exchanger 201 of the heat pump 200.

Besides using an open loop heat pump with a flash apparatus 203 as shown here, it is possible to evaporate at least a part of the water in the heat exchanger 201 of the heat pump 200. If only a part of the water is evaporated in the heat exchanger 201, the partially evaporated water is fed into a phase separator for separating steam from the non-evaporated water. If the phase separator is operated at a lower pressure, additional water may evaporate on entry into the phase separator. If, on the other hand, the whole water is evaporated in the heat exchanger 201, the phase separator can be omitted.

For evaporating water at least partly in the heat exchanger 201, it is necessary that the pressure of the water fed into the heat exchanger 201, is below the boiling pressure at the temperature of the part 13 of the crude product stream 7 fed into the heat exchanger 201. To evaporate water at a higher pressure, the part 13 of the crude product stream 7 may be compressed before being fed into the heat exchanger 201. By compression, the temperature of the part 13 of the crude product stream 7 increases and, hence, the water may have a higher pressure than for being evaporated by heat transfer from the uncompressed top stream. To minimize the energy for compression, the compressor for compressing the part 13 of the crude product stream 7 is arranged between the position at which the part 13 of the crude product stream 7 branches off and the heat exchanger 201.

## Claims

1. A process for producing butyraldehyde, comprising:
(a) feeding propene (1) and a hydrogen and carbon monoxide containing synthesis gas (3) into a reactor (5);
(b) reacting propene, hydrogen and carbon monoxide to obtain an isobutyraldehyde and n-butyraldehyde comprising crude product stream (7);
(c) withdrawing the crude product stream (7) from the reactor (5);
(d) cooling the crude product stream (7);
wherein the crude product stream (7) is cooled in a heat exchanger (201) of a heat pump (200) for producing steam and/or in a heat exchanger (101) of a district heating grid (100).

2. The process according to claim 1, wherein the crude product stream (7) is fed into a vessel (23) for separating gaseous components (27) from crude butyraldehyde (25).

3. The process according to claim 2, wherein the crude butyraldehyde (25) is withdrawn from the vessel (23) and heated in an internal heat exchanger (9) by heat transfer from the crude product stream (7) before being fed into a work-up unit (35).

4. The process according to claim 2 or 3, wherein the gaseous components (27) are heated in an internal heat exchanger (11) by heat transfer from the crude product stream (7) and then recycled into the reactor (5).

5. The process according to any of claims 3 and 4, wherein the crude product stream (7) is cooled in the internal heat exchanger (9) for heating the crude butyraldehyde (25) and/or the internal heat exchanger (11) for heating the gaseous components (27) before being cooled in the heat exchanger (201) of the heat pump (200).

6. The process according to any of claims 1 to 5, wherein the heat pump (200) is a closed loop heat pump or an open loop heat pump.

7. The process according to any of claims 1 to 6, wherein an additional cooler (17) is arranged downstream the heat exchanger (201) of the heat pump (200).

8. The process according to any of claims 1 to 7, wherein the steam (205) is compressed in at least one compressor (207) with at least one compressor stage (207.1, 207.2).

9. The process according to any of claims 1 to 8, wherein a heat transfer medium (103) is heated in the heat exchanger (101) of the district heating grid (100).

10. The process according to claim 9, wherein the heat transfer medium (103) is used as a heat source for heating a heating medium (105) that is distributed in the district heating grid (100).

11. The process according to claim 9 or 10, wherein the heat transfer medium (103) is water.

12. The process according to any of claim 9 to 11, wherein the heating medium (105) is heated in a heat pump by using heat of the heat transfer medium (103) or is heated by heat transfer from the heat transfer medium (103) in a first heat exchanger and subsequent heating in a heater.

13. The process according to any of claims 9 to 11, wherein the heat transfer medium (103) is further heated and then used as heating medium in the district heating grid.
